# EUROPEAN PATENT APPLICATION

(11) **EP 1 742 519 A2**
(43) Date of publication of application: **10.01.2007**
(21) Application number: 06425470.9
(22) Date of filing: 06.07.2006
(51) Int. Cl.: H05H 9/00

(54) **Mobile linac for radiation therapy having in-head oscillator**

(30) Priority: 07.07.2005 IT LT20050003
(71) Applicant: Gatto, Pompilio, 00040 Roma RM (IT)
(72) Inventor: Gatto, Pompilio, 00040 Roma RM (IT)

(57) **Abstract**

Electromedical apparatus for intraoperative radiation therapy by means of electron accelerator, including a radiating head (55), a linear electron accelerator (23), assembled in the radiating head (55), and a RF generator (21) connected to the linear electron accelerator (23). The RF generator (21) too is assembled in the radiating head (55), and is connected with the linear electron accelerator (23) through a rigid waveguide (22).

## Description

### TECHNICAL FIELD

This invention relates to a mobile electromedical apparatus for Intraoperative Radiation Therapy (or "IORT") by means of Linear Accelerator (or "linac") of Electrons.

### PRIOR ART

An electron linac is an evacuated waveguide in which a high-frequency travelling electromagnetic wave is excited, the axial electric field of which accelerates electrons. The phase velocity of the wave is made equal to the velocity of electrons by assembling irises in the guide, which delimitate accelerating coupled resonant cavities. The linac is supplied by a radiofrequency (or "RF") oscillator through a waveguide. A feature of a linac is the *collimation* of the beam, i.e. the parallelism of the trajectories of the electrons it emits.

A mobile "linac for IORT" is a mobile apparatus for applying radiations to produce a destructive effect on a tumour tissue. Electron irradiation has the advantage relative to photon irradiation of penetrating in the tumour tissue in a modulated manner (according to the desired depth), with the exclusion of surrounding healthy tissues. The surgeon after the removal of a tumour fixes the area to be treated with the radiotherapist and selects the energy of the beam with the sanitary physicist to administer the proper dose at a set depth.

A known-art apparatus includes a mobile radiating head containing an irradiation group including a linac. The beam emitted by this one is collimated by means of an applicator and scattered through the interaction with air existing inside the applicator. The applicator includes an upper applicator, directly connected with the radiating head, and a lower applicator, which is arranged in the surgical breach. With the object in view of the treatment, the upper applicator, in an operating stage of *docking* (i.e. of aligning and coupling) is brought in axis with the lower applicator, at a short distance from it, and connected to it through a ring nut.

The radiating head is supported by a "stand", including an articulated arm assembled on a motor mobile bedplate by a coupling which allows its yaw and pitch. The radiating head is assembled to the arm by a coupling which allows its roll and pitch.

The linac is supplied by a RF generator assembly including a cavity magnetron, equipped with a cathode modulation device and connected with the linac through a waveguide.

In known-art apparatuses the irradiation assembly is assembled in the radiating head, and the RF generator assembly is assembled in the arm of the stand, separately from the radiating head, beyond the coupling of this one. The waveguide that connects the linac to the RF generator assembly is a flexible waveguide that steps over the coupling of the radiating head, passing outside the apparatus, and which in operation bends and twists, correspondingly assuming bending and twisting configurations.

Problems are connected with such an "external" flexible waveguide, or "external waveguide".

The flexible waveguide turns out to be the component of the apparatus which wears most. Such a wear is promoted by the mechanical stress which is determined upon it as a consequence of said bending and, still more, twisting per se, and by the fact that in determined positions it goes to scrape against the ceilings, in particular against false ceilings (which are low) of operating theatres. The external guide presents considerable problems in passing through the entry/exit door of operating theatres. The failure rate of the external guide is also increased by the risk that it breaks when one applies a twisting which is accentuated, but required by the type of surgical breach under examination at a given moment.

The external guide heavily limits the movements of the apparatus in translation on the floor (macromovements) as a consequence of the fact that it goes to interfere with ceilings, it being also possible that the apparatus remains blocked. This is a limiting factor not of little account, because it is critical that the operator has ease in displacing the apparatus, in particular he/she can bring it on a side and on the other of an operating bed, to access an surgical breach and bring the radiating head close to it. The external guide, moreover, limits the movements of the head as for roll and pitch (micromovements), in that it "tethers" the head itself. This "tethering" of the head is too a limiting factor of considerable account, because it is critical that the head has ease of displacement for the purposes of the docking, an essential factor to execute the irradiation.

A further problem brought by the external guide is that when this one bends or twists, the frequency of the wave guided in it alters, the frequency of oscillation of the magnetron so going out of coincidence with the resonance frequency of the linac.

### DISCLOSURE OF THE INVENTION

Therefore, in view of the problems of the apparatuses having an external waveguide, this invention puts itself the object of solve them, by eliminating such a guide.

This object is achieved by this invention by displacing the RF generator assembly into the radiating head, connecting it there to the irradiation assembly, advantageously through a (short) rigid waveguide or directly, so the frequency of oscillation of the RF generator being automatically kept coincident with the resonance one of the linac in a highly reliable manner.

It is an advantage of the inventive apparatus the great mobility of it, it being able to pass through a door even less high than 2.20 m.

It is an advantage of this invention that by virtue of it so a close approaching is realized of the radiating head to a tumour zone, that a very fine position adjustment is possible of the head itself, whereby the docking turns out to be enormously facilitated.

It is an advantage of this invention that by it the cost turns out to be eliminated of the external guide, the cost turns out to be diminished of the electronics of the apparatus because the electronics is no more present for managing the external guide and the cost turns out to be diminished for the assembly of the apparatus.

Moreover, this invention takes its steps from ascertaining that tumours exist, like skin melanomas, which present themselves in zones having a highly uneven contour, the irradiation of which, if carried out to a poor precision, such as by known-art apparatuses making use of collimating applicators, involves the irradiation of a lot of healthy surrounding tissue, and that, therefore, the exigency is strongly felt of being able to carry out the irradiation of a tumour in an extremely precise manner.

Therefore, this invention poses itself the further object of satisfying such an exigency by providing an apparatus with which it turns out to be possible to carry out a dot-like (point-to-point) treatment of a tumour zone, bringing the radiating head to scan the zone faithfully within the contour of it.

Such an object is reached by equipping an inventive apparatus with a self-focusing linac. In fact the restriction of the electron beam emitted by a self-focusing beam, in conjunction with the very fine position adjustment of the radiating head that can be achieved by an inventive apparatus allows a very fine intervention control to be achieved.

It is an advantage of the apparatus having a self-focusing linac that the applicator is not used with it and therefore the difficulties of the docking are no more present.

Therefore, it is the subject of this invention an electromedical apparatus according to annexed Claim 1.

Preferred embodiments are set forth in dependent claims.

### BRIEF DESCRIPTION OF THE DRAWINGS

This invention will be fully understood based on the following detailed disclosure of an exemplifying, absolutely not restricting embodiment thereof, referring to the annexed drawings, wherein:
- FIGURE 1 represents a block diagram of an inventive apparatus;
- FIGURE 2 represents a view of an inventive apparatus, arranged in proximity to an operating bed.

### BEST WAY OF REALIZING THE INVENTION

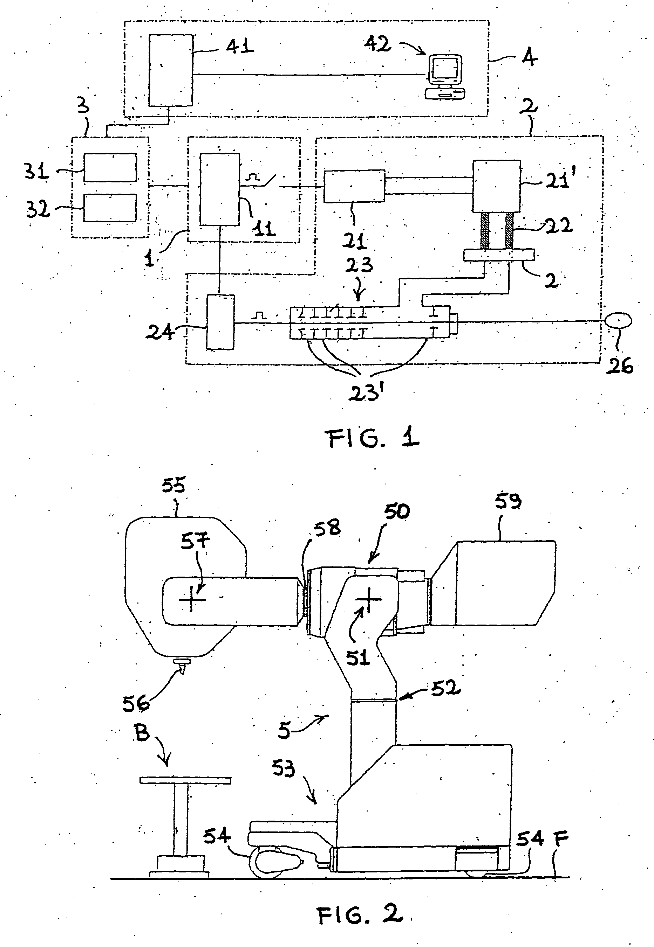

Referring to FIGURE 1, an inventive apparatus includes a high-voltage generator assembly 1, a RF generation and irradiation unit 2, a stand management system 3 and a control and processing system 4, in functional communication therebetween.

High-voltage generator assembly 1 includes a power-supply HVPS 11, a delay line, a pulse transformer, and power cables for its external connection.

RF generation and irradiation unit 2 as integral is preceptive. The RF generation part includes a cavity magnetron 21, constituting an oscillator, with a protection isolator 21'. The irradiation part includes an electron linac 23, formed of a plurality of cavities 23', connected with a cathode modulation device 24, and to magnetron 21 through a (short) rigid waveguide 22 and to a waveguide connector 25, and an electron beam output 26.

It is envisaged that linac 23 is "self-focusing", i.e. that the focusing of the electron beam takes place as a consequence of the constitution of the linac and not by external magnets. For this purpose accelerating cavities 23' can be of increasing lengths from the first to the fifth cavity and thereafter constant. One can make the electrons to pass through the cavities successive to the fifth one after a RF peak, without a further focusing.

Stand management system 3 includes a cooling subsystem 31 and a motor subsystem 32. Control and processing system 4 includes an electric power supply 41 and a processor 42.

Referring to FIGURE 2, an inventive apparatus, placed at an operating bed B, includes a "stand" 5 including an articulated arm 50 assembled mobile in pitch 51 and in yaw 52 on a motor 32 bedplate 53, mobile in translation 54 on floor F. A radiating head 55 having an electron beam output diaphragm, represented with an upper applicator 56 assembled thereon, is assembled at an extremity of the arm in a completely mobile manner (across 180°) in pitch 57 and in a completely mobile manner (across 360°) in roll 58.

Radiating head 53 includes, preceptively in addition to the linac, the RF generation assembly, including the oscillator, the isolator and the waveguide, whilst in a separate section 59 of the arm, high-voltage generator assembly 1 is assembled.

This invention has been described and illustrated referring to a specific embodiment thereof, but it is to be expressly understood that variations, additions and/or omissions can be made, without departing from the relevant scope of protection, which only remains restricted by the annexed claims.

## Claims

1. Electromedical apparatus for intraoperative radiation therapy by means of linear electron accelerator, including a radiating head (55); means for linear acceleration of electrons (23), assembled in said radiating head (55), and means for generating electromagnetic waves (21) connected to said means for linear acceleration of electrons (23), **characterized in that** said means for generating electromagnetic waves (21) are assembled in said radiating head (55).

2. Apparatus of Claim 1 wherein said means for linear acceleration of electrons (23) are connected with said means for generating electromagnetic waves (21) through a rigid waveguide (22).

3. Apparatus of Claim 1, wherein said means for linear acceleration of electrons (23) are a self-focusing linear electron accelerator.
